# EUROPEAN PATENT APPLICATION

(11) **EP 4 039 269 A1**
(43) Date of publication of application: **10.08.2022**
(21) Application number: 21155393.8
(22) Date of filing: 05.02.2021
(51) Int. Cl.: A61K 38/18, A61P 27/02

(54) **NGF ISOFORM FOR USE IN THE TREATMENT OF OCULAR PATHOLOGIES**

(71) Applicant: Dompe' Farmaceutici S.P.A., 20122 Milan (IT)
(72) Inventor: ARAMINI, Andrea, 67100 L'Aquila (IT); AMENDOLA, Piergiorgio, 80131 Napoli (IT); SIRICO, Anna, 80131 Napoli (IT); D'ANNIBALLE, Gaetano, 67100 L'Aquila (IT); CATTANI, Franca, 67100 L'Aquila (IT); ALLEGRETTI, Marcello, 67100 L'Aquila (IT); MANTELLI, Flavio, 67100 L'Aquila (IT)
(74) Representative: Mauri, Elisabetta Maria Ester

(57) **Abstract**

The present invention relates to the field of treatment of ocular pathologies by administration of a NGF, which comprises more than 50% by weight of the NGF isoform of SEQ ID NO 1. Said NGF is particularly useful in the treatment of ocular pathologies where the proliferation and survival effect of NGF is desired and where the proapoptotic effect of p75^{NTR} is detrimental.

## Description

### FIELD OF THE INVENTION

The present invention relates to the field of treatment of ocular pathologies, in particular of treatment of ocular pathologies by administration of a specific isoform of NGF.

### STATE OF THE ART

The nerve growth factor (NGF) is a member of the family of evolutionarily well-conserved neurotrophin growth factors, which also includes brain-derived neurotrophic factor (BDNF), neurotrophin-3 (NT3) and NT4/5.

It exerts its activity by interacting with two structurally unrelated cell surface receptors: the high affinity receptor tyrosine kinase A (TrKA) and the low affinity p75 neurotrophin receptor (p75^{NTR}).

It has been shown that these two receptors mediate complex and often opposite effects of NGF on cells.

TrKA is selective for NGF and triggers different signalling pathways that promote cell survival, proliferation and differentiation, such as PI3 kinase, Ras/extracellular signal-regulated kinases (ERK), Akt 1 and protein kinase C (PKC) (Kaplan et al., J Neurobiol 1994, 25(11 ):1404-17; Clewes et al., J Biochem 2008, 107: 1124-1135). Furthermore, the activation of TrKA inhibits apoptotic signaling in cells, for example by inhibiting capsase 3 (Nguyen T LX, Eperimental and Molecular Medicine 2010, 42 (8): 583-595).

The p75 neurotrophin receptor (p75^{NTR}) belongs to the tumour necrosis factor receptor superfamily and binds non-specifically all neurotrophins. Contrary to what observed when binding to the TrKA receptor, NGF binding to p75^{NTR} induces apoptosis through the activation of a number of intracellular mediators similar to those activated by death receptors, such as tumor necrosis factor (TNF) and Fas receptors, including TNF receptor associated factors (TRAFs), nuclear Kappa B (NFₖB), capsases and p53 (Harada C et al., Developmental Biology 2006, 290: 57-65; Aloyz R S et al., The Journal of Cell Biology 1998, (143) 6: 1691-1703).

Thus, the final activity of NGF on cells is significantly dependent on the level of expression and on the activation of the above two receptors. Therefore, changes in the ratio of the two receptors can alter the balance between protective and deleterious effects induced by NGF, thus modifying the final activity of NGF on cells, with the predominance of either proliferative and survival effects mediated by TrKA or of apoptotic responses mediated by p75^{NRT} (Frade at al., Nature 1996; 383: 166-168; Yoon So et al., J Neuroscience 1998; 18: 3273-3281).

The affinity of NGF to p75^{NTR} is lower than to TrKA, but its cell type distribution is wider than that of TrKA.

Both NGF receptors are broadly expressed in both the anterior and posterior segments of the eye, including cornea, conjunctiva, limbal epithelium, retina and optic nerve and NGF has been demonstrated to have a key role in both eye physiology and pathology, modulating processes such as cell survival, proliferation, differentiation and apoptosis (Garcia et al., Cytokine & Growth Factors Reviews 2017, 34: 43-57; Sornelli et al., Molecular Vision 2010; 16:1439-1447; Di Girolamo et al., J Cell Mol. Med 2008, 12(6B): 2799-2811). Several experimental studies demonstrated that TrKA stimulation promotes RGCs survival after ischemic injury, optic nerve transection, and ocular hypertension. (Carmignoto et al., Journal of Neuroscience 1989, 9(4): 1263-1272.; Chakrabarti et al., Brain Research 1990; 523:11-15; Siliprandi et al., Invest Opthalmol Vis Sci 1993, 34 (12): 3232-3245; Haamedi et al., The Journal of Comparative Biology 2001, 431: 397-404; Harada et al., Developmental Biology 2006 290: 57-65; Coassin et al., Graefes Arch Clin Exp Ophthalmol 2008, 246:1743-1749).

Notwithstanding the above, the effects of NGF in the visual system are complex and they are not univocal, depending upon the cellular context and the cellular distribution and level of expression of each NGF receptor. For example, in the retina, RGCs express TrKA and glial cells express p75^{NTR}. A study in an animal model of glaucoma showed that a selective agonist of the pro-survival TrKA receptor was effective at preventing RGC death, while neither NGF nor an antagonist of the pro-apoptotic p75 receptor protected RGCs. (Shi et al., Developmental Neurobiology 2007, 67(7): 884-94).

A subsequent study showed enhanced survival of axotomized RGCs in pharmacological inhibition of p75NTR or in p75NTR knockout mice. In addition, a combination of NGF or TrKA agonists with p75NTR antagonists further potentiated RGC neuroprotection in vivo (Lebrun-Julien F, Molecular and Cellular Neuroscience. 2009, 40(4): 410-420).

In summary, several data support the hypothesis that NGF can exert neuroprotective effects when binding on RGCs TrKA receptor while acting on glial cells p75^{NTR} antagonizes this effect (Wang H et al., BioMed Research International 2014, Article ID 759473).

These controversial findings can be explained by the observation that NGF has different actions on RGCs because of different relative expression of TrKA and p75^{NTR} in the retina upon different circumstances, thereby the failure of NGF trophic support might be associated with the progressive up-regulation of p75^{NTR} in relation to TrKA (Coassin et al., Graefes Arch Clin Exp Ophthalmol 2008, 246:1743-1749; Mohamed R et al J Clin Exp Ophthalmol. 2015; 6(5); doi:10.4172/2155-9570.1000483).

This evidence is confirmed by studies that demonstrate that p75^{NTR} selective activation induces RGC death in the normal retina, and accelerates RGC death in diseased eyes, while selective TrKA agonists protect RGCs in chronic and acute neurodegeneration (Bai Y et al., Journal of Biological Chemistry 2010, 285 (50): 39392-39400; ZhiHua Shi et al., Developmental Neurobiology (2007), 67 (7): 884-894).

Some studies have also demonstrated that in the retina, TrKA is mainly expressed in retinal ganglion cells (RGCs) while p75^{NRT} in Muller and glial cells (Harada C et al., Developmental Biology 2006, 290: 57-65) and that the activation of p75^{NRT} in glia is able to trigger neurotoxic pathways that counterbalance the protective effect of TrKA activation on RGCs.

A further reason for the inconsistency of the data obtained may also be due to the use of different routes or regimens of administration of the protein. In fact, the concentration at which the protein is administered seems to influence the response to NGF, with higher concentrations resulting in a higher activation of p75^{NTR}. Therefore, administration routes or regimens that require high concentrations of NGF may be less effective in inducing survival activity of NGF than low concentrations.

Other potential therapeutic applications of NGF in other conditions of the eye are based on the prosurvival and trophic effect of this neurotrophin and therefore its efficacy is highly dependent on the balance between TrKA and p75^{NTR} activation.

In particular, evidence shows that the antiaptototic and trophic effects of NGF may be useful in:
- preventing corneal graft rejection in corneal transplantation (Gong N et al., Invest Opthalmol Vis Sci, 2007, 48 (3): 1043-1052);
- preserving and expanding limbal epithelial progenitor cells (Lambiase et al., Invest Opthalmol Vis Sci 2012, 53(13): 8280-8287, Mason SL, et al. Invest Ophthalmol Vis Sci. 2016; 57: 3708-3713, Touhami A et al., Invest Ophthalmol Vis Sci. 2002, 43: 987-994);
- in the treatment of corneal and conjunctival diseases, including phototoxic keratopathy (Rocco ML et al., Graefes Archive for Clinical and Experimental Ophthalmology 2018, 256: 729-738), corneal dystrophies and degenerations and corneal ulcers (Lambiase et al., Invest Opthalmol Vis Sci 1998, 39: 1272-1275; Lambiase, et al., N Engl J Med 1998, 338: 1174-1180; Bonini et al., Ophthalmology 2000,107: 1347-1351; Lambiase et al., Invest Ophthalmol Vis Sci 2000, 41: 1063-1069: Blanco-Mezquita et al., Invest Ophthalmol Vis Sci. 2013, 54(6): 3880-90: You L et al., Invest Ophthalmol Vis Sci. 2000; 41(3): 692-702; Sornelli F, et al., Mol Vis. 2010, 29;16: 1439-47), keratoconjunctivitis sicca (Coassin et al., Graefes Arch Clin Exp Ophthalmol 2008, 246:1743-1749), retinal diseases including retinal detachment (Sun et al., Ophthalmologica 2008, 222: 58-61), diabetic retinopathy (Ali et al., Diabetes, 2008, 57: 889-898), retinal neurodegeneration and/or ischemia (Xien et al., Exp Eye Res 2014, 125: 156-63), phototoxic retinopathy (Rocco et al., Graefes Arch Clin Exp Ophthalmol 2018, 256: 729-738; Garcia et al., J Neurochem 2014, 131(3): 303-13), epiretinal membrane (Minchiotti et al., Retina 2008, 28(4): 628-37), macular hole (Zhang et al., BMC Ophthalmol 2019, 19(1): 130), macular degeneration (Lambiase et al., Ann Ist Super Sanità 2009, 45 (4): 439-442) and optic neuropathies (Mesentier-Louro et al., Mol Neurobiol. 2019, 56(2): 1056-1069; Guo et al Sci Rep 2020, 10: 3375).

However, the activation of the p75^{NTR} receptor by NGF makes the *in vivo* effect of NGF in the above pathologies difficult to predict.

A number of different rhNGF variants of 117 and 118 aminoacids have been identified when the protein is expressed as a 120-aminoacid sequence in Chinese Hamster Ovary cells, due to partial enzymatic digestion by trypsin and/or carboxypeptidase. These variants have been analysed and found to be equipotent in both the chick dorsal root ganglion cell survival and rat pheochromocytoma neurite extension assays (Shmelzer et al., J Neurochem 1992, 59(5):1675-83).

### SUMMARY OF THE INVENTION

The Applicant has undertaken studies aimed at clarifying the somewhat controversial data in the literature on NGF activity in the eye.

These studies have shown that commercially available NGFs can include different isoforms of NGF, characterised by aminoacid sequences differing only in length, either alone or in admixtures.

Furthermore, the present inventors have surprisingly found that these isoforms of NGF have different ability to activate pathways induced by the TrKA or p75^{NTR} receptors.

In particular, the present inventors have found that the NGF isoform having the 118 aminoacid sequence of SEQ. ID NO.1 predominantly activates TrKA-mediated pathways and inhibits apoptotic pathways, while isoforms having 120, 117 or119 aminoacid sequences of SEQ ID NO.2, 3 and 4, respectively, have a higher ability to activate p75^{NTR}-dependent apoptotic pathways. These findings, relating to the structural inhomogeneity of the NGF protein present in commercial preparations of the protein and to the different receptor selectivity of the NGF isoforms, can help to understand the inconsistencies found in the literature regarding the therapeutic applications of NGF.

In view of the above, NGF isoform of SEQ ID NO 1 is particularly useful in the treatment of pathologies where the effects of NGF on proliferation and survival are desired and where the proapoptotic effect of p75^{NTR} is detrimental.

Accordingly, a first object the present invention is a NGF for use in the prevention and/or treatment of an ocular pathology selected from retinopathies, preferably selected from diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane; limbal stem cell deficiency; corneal pathologies, preferably selected from keratoconus, phototoxic keratopathy, persistent epithelial defects, corneal ulcers, corneal dystrophies and degeneration and keratoconjunctivitis sicca; conjunctival pathologies; optic neuropathies, preferably selected from glaucoma, ischemic, degenerative, traumatic, inherited and congenital optic neuropathies; and in the prevention of allograft rejection in corneal transplantation, in which said NGF comprises more than 50% by weight of the NGF isoform of SEQ ID NO 1 relative to the total weight of all NGF isoforms possibly comprised in said NGF.

A second object of the present invention relates to a pharmaceutical composition comprising a NGF in a therapeutically effective amount, in which said NGF comprises more than 50% by weight of the NGF isoform of SEQ ID NO 1 relative to the total weight of all NGF isoforms possibly comprised in said NGF and at least one pharmaceutically acceptable excipient, for use in the treatment of an ocular pathology selected from retinopathies, preferably selected from diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane; limbal stem cell deficiency; corneal pathologies, preferably selected from keratoconus, phototoxic keratopathy; persistent epithelial defects, corneal ulcers, corneal dystrophies and degeneration and keratoconjunctivitis sicca; conjunctival pathologies; optic neuropathies, preferably selected from glaucoma, ischemic, degenerative, traumatic, inherited and congenital optic neuropathies; and in the prevention of allograft rejection in corneal transplantation.

A third object of the present invention relates to a method of treating an ocular pathology selected from retinopathies, preferably selected from diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane; limbal stem cell deficiency; corneal pathologies, preferably selected from keratoconus, phototoxic keratopathy, persistent epithelial defects, corneal ulcers, corneal dystrophies and degeneration and keratoconjunctivitis sicca; conjunctival pathologies; optic neuropathies, preferably selected from glaucoma, ischemic, degenerative, traumatic, inherited and congenital optic neuropathies; and in the prevention of allograft rejection in corneal transplantation, comprising administering to the subject a NGF in a therapeutically effective amount, in which said NGF comprises more than 50% by weight of the NGF isoform of SEQ ID NO 1 relative to the total of all NGF isoforms possibly comprised in said NGF.

### BRIEF DESCRIPTION OF THE FIGURES

Figure 1 shows the expression of the NGF receptors TrKA and p75^{NTR} on the cell membrane of PC12, HCEC and RPE cells, measured as described in Example 2. The data are represented as percentage of positive cells expressing the relevant receptor, measured by flow cytometry.
Figure 2 shows the total number of proteins upregulated and downregulated in each cell line by treatment with each NGF, measured as described in Example 2 and represented as Venn diagram. In details, Figure 2A) shows the Venn diagram for RPE cells, Figure 2B) shows the Venn diagram for HCEC cells and Figure 2C) shows the Venn diagram for PC12 cells.
Figure 3 shows early (30 minutes, Figure 3A) and late (24 hours, Figure 3B) caspase 3/7 activation in HCEC cells not treated (NT), treated with PBS (PBS), formulation buffer (FB), rhNGF-118 (rhNGF-118) or rhNGF-1 (rhNGF-1), as described in Example 3a. The results are expressed as the ratio between green area (caspase activation) and phase area (cell confluence) of seven independent experiments. Student's T test was calculated, *p-value <0.05, ns=not significant.
Figure 4 shows early (30 minutes, Figure 4A) and late (24 hours, Figure 4B) caspase 3/7 activation in RPE cells not treated (NT), treated with PBS (PBS 1X), formulation buffer (FB), rhNGF-118 (rhNGF-118) or rhNGF-1 (rhNGF-1), as described in Example 3a. The results are expressed as fold of change over not treated (NT) of the ratio between green area and phase area of five independent experiments. Student's T test was calculated, *p-value<0.05.

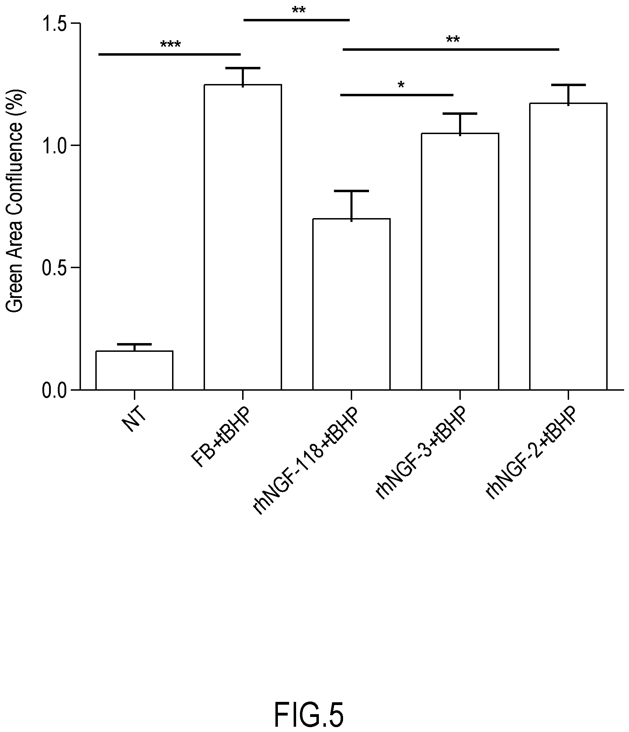

Figure 5 shows caspase 3/7 activation in RPE cells not treated (NT) or treated for 24 hours with 10µM tBHP in the presence of formulation buffer (FB + tBHP), 50 ng/ml of rhNGF118 (rhNGF-118 + tBHP), 50 ng/ml of rhNGF-2 (rhNGF-2 + tBHP) or 50 ng/ml of rhNGF-3 (rhNGF-3+ tBHP), as described in Example 3b.i). Data are presented as percentage of green area confluence (caspase activation) of three independent experiments. Student's T test was calculated, *p-value<0.05, **p-value<0.005, ***p-value<0.0005. Moreover, one-way ANOVA, Bonferroni test, showed a statistical significance of tBHP versus NT, of rhNGF-118 versus tBHP and of rhNGF-2 versus rhNGF-118 (not shown).

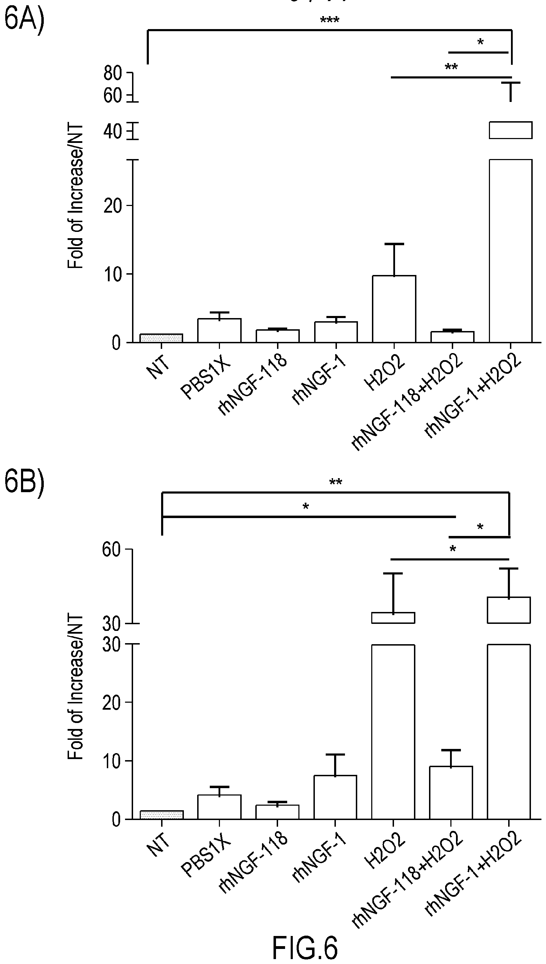

Figure 6 shows early (30 minutes, Figure 6A) and late (24 hours, Figure 6B) caspase 3/7 activation in RPE cells not treated (NT) or treated with PBS (PBS 1X), 50 ng/ml rhNGF-118 (rhNGF-118), 50 ng/ml rhNGF-1 (rhNGF-1), 100µM H₂O₂ alone (H₂O₂) or in the presence of 50 ng/ml rhNGF-118 (rhNGF-118+ H₂O₂) or 50 ng/ml rhNGF-1 (rhNGF-1 + H₂O₂), as described in Example 3b.ii). Data are presented as fold of increase over not treated cells (NT) of total green fluorescence (caspase activation) of four independent experiments. Student's T test was calculated, *p-value<0.05, **p-value<0.005, ***p-value<0.0005. Moreover, after 30 minutes one-way ANOVA, Bonferroni test, showed a statistical significance of H₂O₂ in the presence of rhNGF-1 versus NT, versus H₂O₂ alone and versus H₂O₂ in the presence of rhNGF-118; while after 24 hours a statistical significance was observed of H₂O₂ in the presence of rhNGF-1 versus NT (not shown).
Figure 7 shows neurite growth induced by two different concentrations of rhNGF-118 or rhNGF-1, measured as described in Example 4.
Figure 8 shows the expanded reverse phase HPLC chromatogram of rhNGF-118.
Figure 9 shows the expanded reverse phase HPLC chromatogram of rhNGF-1.
Figure 10 shows the expanded reverse phase HPLC chromatogram of rhNGF-2.
Figure 11 shows the expanded reverse phase HPLC chromatogram of rhNGF-3.

### DETAILED DESCRIPTION OF THE INVENTION

A first object the present invention is a NGF for use in the treatment of a pathology selected from retinopathies, corneal pathologies, optic neuropathies, conjunctival pathologies, limbal stem cell deficiency and in the prevention of allograft rejection in corneal transplantation, in which said NGF comprises more than 50% by weight of the NGF isoform of SEQ ID NO 1 relative to the total weight of all NGF isoforms possibly comprised in said NGF.

In the present context, for "NGF isoform" it is meant any of two or more functionally active NGF proteins having different aminoacid sequences, wherein such sequences differ only in their length. When referring to a NGF isoform, all forms of NGFs having the relevant aminoacid sequence are possibly included, comprising NGFs having the relevant sequence and possibly carrying post-translational modifications, such as oxidation, glycation or glycosylation.

In a preferred embodiment, said NGF isoforms possibly comprised in said NGF are selected from NGF isoforms of SEQ ID NO 1, 2, 3, 4 or admixtures thereof.

Preferably, said retinopathies are selected from diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane.

Preferably, said corneal pathologies are selected from keratoconus, phototoxic keratopathy, persistent epithelial defects, corneal ulcers, corneal dystrophies and degeneration, and keratoconjunctivitis sicca.

Preferably, said optic neuropathies are selected from glaucoma and ischemic, degenerative, traumatic, inherited and congenital optic neuropathies.

According to a particularly preferred embodiment, the above pathology is selected from glaucoma, diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane, more preferably it is glaucoma.

The NGF for use according to the present invention preferably consists in a high purity NGF isoform of SEQ ID NO 1.

Preferably, the NGF for use according to the present invention comprises at least 80%, more preferably at least 90%, even more preferably at least 95%, even more preferably at least 98%, even more preferably at least 99%, even more preferably 100% by weight of the NGF isoform of SEQ ID NO 1 relative to the total weight of all NGF isoforms possibly comprised in said NGF.

According to a preferred embodiment, the NGF for use according to the present invention comprises at least 80%, more preferably at least 90%, even more preferably at least 95%, even more preferably at least 98%, even more preferably at least 99%, even more preferably 100% by weight of the NGF isoform of SEQ ID NO 1 relative to the total weight of all NGF isoforms possibly comprised in said NGF, in which said NGF isoforms possibly comprised in said NGF comprise NGF isoforms of SEQ ID NO 1, 2, 3, 4 or admixtures thereof.

Preferably, the NGF for the present use comprises NGF isoforms of SEQ ID NO 2, 3, 4 or their admixtures in total weight lower than 20%, more preferably lower than 10%, even more preferably lower than 5%, even more preferably lower than 2%, even more preferably lower than 1% by weight, relative to the total weight of all NGF isoforms possibly comprised in said NGF.

According to a preferred embodiment, said NGF isoform of SEQ ID NO 1 is not used in combination with NGFs having SEQ ID NO 2, 3 or 4.

In a preferred embodiment, said NGF isoform of SEQ ID NO 1 does not include more than 15%, preferably more than 10% or than 5% by weight in total of post-translational modifications.

In a preferred embodiment, said NGF isoform of SEQ ID NO 1 does not include any post-translational modification, namely it only consists of non-modified aminoacids. The NGF for the present use may comprise other impurities, in total amount preferably lower than 20% by weight, more preferably lower than 10%, even more preferably lower than 5% by weight relative to the NGF total weight. With other impurities, compounds different from NGF isoforms and their post-translational modifications are meant.

Preferably, the above NGF isoform of SEQ ID NO 1 is a recombinant human NGF. This may be manufactured for example in *E.Coli* according to the process described in WO2000/022119 and WO2013/092776, using an expression vector incorporating the sequence of the proNGF mutant SP174-101 (SEQ 10 NO: 5 of WO2013/092776).

Preferably, the above NGF for use according to the first aspect of the invention is administered in the form of a pharmaceutical composition for ophthalmic use.

The exact dose and regimen for administration of the present NGF in the treatment or the prevention of the above pathologies will depend upon many factors, such as for instance the route of administration and the severity of the disease of the individual receiving treatment.

Said pharmaceutical composition comprises the above-described NGF and one or more ophthalmologically acceptable excipients.

An "ophthalmologically acceptable excipient" is an inert excipient which allows delivery of a medicament to the eye and/or eyelids, to treat an ocular disease or condition without deleterious effects on the eye.

According to an embodiment, said ophthalmic composition may be a liquid, eye drop composition for topical administration to the anterior segment of the eye.

Said liquid composition may be in form of a solution, emulsion, or suspension. Said liquid composition may include micelles. In one embodiment, the liquid composition is an aqueous composition.

Preferably, the liquid composition is an aqueous eye drop composition.

Preferably, said liquid composition comprises ophthalmologically acceptable excipients selected from ophthalmologically acceptable viscosity enhancers, penetration enhancers, buffering agents, osmolarity regulators, preservatives and surfactants.

Viscosity enhancers have the function to increase viscosity of the composition and to improve its retention in the conjunctival sac and are preferably selected from cellulose derivatives, preferably hydroxymethylcellulose, hydroxyethylcellulose, hydroxypropylmethylcellulose, methylcellulose; polyvinylpyrrolidone and gelling agents, preferably gellan, xanthan gum and carbopol-974.

Penetration enhancers have the function of enhancing drug permeability across ocular membranes and are preferably selected from cyclodextrins, chelating agent, crown ethers, bile acids and bile salts.

Buffering agents have the function of providing and maintaining the correct pH of the formulation to be compatible for use in the eye, preferably at a pH comprised between 6 and 8. The preferred buffer is phosphate buffer, but other buffers capable of maintaining the pH within the desired range, especially buffers suitable for ophthalmic use, are also included.

Osmolarity regulators are salts able to make the liquid composition isotonic with ocular fluids. The preferred salt is sodium chloride (NaCl) but other biologically acceptable salts may be used, such as for instance potassium chloride (KCI), calcium chloride (CaCl₂) and magnesium chloride (MgCl₂) and their admixtures.

Preservatives inhibit microbial activity. Suitable preservatives include for instance quaternary ammonium compounds such as benzalkonium chloride, cetyltrimethylammonium bromide and cetylpyridinium chloride.

Surfactants have the function of making the composition stable and of reducing or preventing NGF adsorption to various surfaces of the container and are preferably selected from polysorbates such as Tweeny 80, poloxamers such as Pluronics F68 or proteins such as serum albumin.

Said liquid, eye drop composition can be part of a kit comprising the composition, a container for holding the composition and a drop dispenser.

The NGF aqueous composition may comprise a sufficient amount of biologically acceptable salts to provide the correct fluid tonicity and to maintain the NGF in solution. Other additives commonly used in pharmaceutical aqueous compositions and known to the technical expert, such as sugars, sugar alcohols, aminoacids, cellulose-derivatives, polyethylene glycols, may be present in the NGF aqueous composition.

The NGF aqueous composition comprises water in an amount sufficient to achieve the appropriate concentration of composition components.

The liquid composition comprises NGF in therapeutically effective concentrations. Preferably, in the liquid composition, the above-described NGF is present at concentrations ranging from about 0.0001% to about 0.5% w/v, more preferably from about 0.001% to about 0.1% w/v, most preferably of about 0.002% w/v of the aqueous composition.

According to an embodiment, the present composition is a composition suitable for administration to the posterior segment of the eye, preferably by intravitreal injection or surgical implantation. The NGF for use according to the present invention is particularly advantageous for this type of administration since it requires the use of higher concentrations of NGF. At these concentrations, the presence of isoforms different from the NGF isoform of SEQ ID NO 1 may result in a marked increase in p75^{NTR} activation and, as a consequnce, more relevant side effects. Hence, for this application, NGF preferably comprises NGF isoforms of SEQ ID NO 2, 3, 4 or their admixtures in total weight lower than 5%, more preferably lower than 2%, even more preferably lower than 1% by weight, relative to the total weight of all NGF isoforms possibly comprised in said NGF.

Preferably, according to this embodiment, the composition for use according to the invention is administered, by intravitreal injection or implantation, to the retina, sclera, posterior chamber, vitreous chamber, subretinal space, suprachoroidal segment of the eye.

Preferably, according to this embodiment, NGF is present in the composition at a concentration ranging from about 0.01% to about 0.1% w/v, more preferably from about 0.02% w/v to about 0.05% w/v, most preferably from about 0.03% to about 0.04% w/v. According to particularly preferred embodiment, said ophthalmic composition is a controlled relaease composition for intravitreal administration into the eye.

According to this embodiment, the present composition can be in form of polymer microparticles, wherein said polymer is preferably a biodegradable or water soluble polymer, having the property of gradually releasing the above described NGF to the eye. The invention will be further described in the following examples, which do not limit the scope of the invention described in the claims.

### EXPERIMENTAL PART

### Example 1- Characterisation of different rhNGFs

### a) Materials

Recombinant human NGF (rhNGF) having an aminoacid sequence of 118 aminoacids (SEQ.ID.NO:1, hereinbelow rhNGF-118) was manufactured in E. *Coli* according to the process described in WO2000/022119 and WO2013/092776, using an expression vector incorporating the sequence of the proNGF mutant SP174-101 (SEQ ID NO: 5 of WO2013/092776).

Three different commercial rhNGFs were purchased:
- rhNGF-1: recombinant human NGF from R&D Systems Inc (product code:256-GF/CF), manufactured in a mouse myeloma cell line.
- rhNGF2: recombinant human NGF from Active Bioscience (product code 1745.955), manufactured in CHO cells.
- rhNGF3: recombinant human NGF from Sino Biological (product code: 11050-HNAC), manufactured in CHO cells.

### b) HPLC Analysis of the different rhNGFs

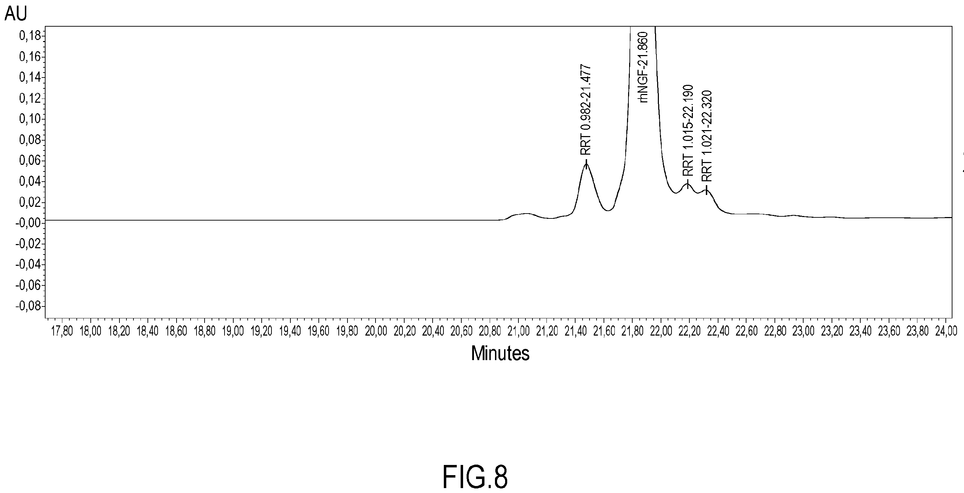

Samples of the above rhNGFs from different origins were analysed by reverse phase HPLC-UV, reverse phase UHPLC-MS for determining the molecular weights and by reverse phase HPLC-MS for peptide mapping. The results of the HPLC analyses are shown in Figures 8 to 11 and reported in Tables 1 to 5 below.

### Reverse phase HPLC-UV analysis

A Waters HPLC system including a volume pumping gradient system, a cooled sample injection device and an UV detector was used for this analysis. The analytical separation was performed using a Phenomenex column, model Jupiter C4, 300 A, 250x4.6 mm (5 µm particle size).

A gradient elution was carried out with a mobile phase consisting of water and acetonitrile, both containing 0.05% of trifluoroacetic acid (TFA). The flow rate was 1 mL/min, the column temperature was maintained at 37°C and the wavelength set at 220 nm.

This procedure was applied in parallel for rhNGF-118 and the commercial rhNGFs described above.

In details, rhNGF samples to be tested were thawed at room temperature and treated according to the instructions provided by suppliers.

Before injection into the HPLC system, all samples were diluted in Formulation Buffer (Phosphate buffer 50mM, NaCl 100 mM pH 7.2) to a final concentration ranging from 0.3 to 0.1 mg/mL.

### UHPLC-MS analysis

A UHPLC system coupled to an Orbitrap QExactive Mass Spectrometer (Thermo Scientific), equipped with a Heated Electrospray Ionization Source (HESI) was used for this analysis.

The analytical separations were performed using a Waters column, model Acquity UPLC protein BEH C4 300A, 100 x 2.1 mm (1.7 µm particle size). A gradient elution was carried out with a mobile phase consisting of water and acetonitrile, both containing 0.05% of trifluoroacetic acid (TFA). The flow rate was 0.3 mL/min and the column temperature was maintained at 33°C. The characterization of intact proteins was performed on mass spectrometry data using the Biopharma Finder Software (Thermo Scientific).

This procedure was applied in parallel for rhNGF-118 and the commercial rhNGFs described above.

In details, NGF samples were thawed at room temperature and treated according to the instructions provided by suppliers. Before injection into the UHPLC system, all samples were diluted in Formulation Buffer to a final concentration ranging from 0.3 to 0.1 mg/mL.

### Reverse phase HPLC-MS for peptide mapping of rhNGFs of different origin

A HPLC system coupled to an Orbitrap QExactive Mass Spectrometer (Thermo Scientific), equipped of a Heated Electrospray Ionization Source (HESI) was used for this analysis.

The analytical separations were performed using a Phenomenex column, model Jupiter C18 300A, 250 x 2.1 mm (5 µm particle size). A gradient elution was carried out with a mobile phase consisting of water and acetonitrile, both containing 0.1% of trifluoracetic acid. The flow rate was 0.2 mL/min and the column temperature was maintained at 53°C. This procedure was applied in parallel for rhNGF-118 and the commercial rhNGFs described above.

In details, NGF samples were thawed at room temperature and treated according to the instructions provided by suppliers.

A total of 300 µg of each NGF sample was precipitated with trichloroacetic acid (TCA) and centrifugated at °4C for 20 minutes. The samples were resuspended and denaturated with guanidine-HCI 5.92M and Ammonium Bicarbonate 100 mM pH 7.8. Reduction was achieved by the addition of dithiothreitol (DTT) 50mM followed by incubation at 56°C for 90 min. Alkylation was performed by adding iodoacetamide (IAA) 75mM following by incubation at room temperature for 30 min, in the dark. Quenching of the excess of IAA was performed by the addition to the samples of the solution of 50mM of DDT followed by incubation at 37°C for 30 min. The enzymatic digestion was carried out adding trypsin (mass ratio 1/17.5 w/w of trypsin/protein) and incubating overnight at 37°C (about 18 hours). After incubation the samples were centrifugated and analyzed. The peptide identification was performed on MS/MS data using the Biopharma Finder Software (Thermo Fisher).

The reverse phase HPLC chromatograms are shown in Figures 8-11 while the main peaks of the corresponding HPLC tabulates are reported in Tables 2 to 5.

As can be seen, the expanded chromatogram for rhNGF-118 (Figure 8) and the Table 2 show a main peak at a retention time (RT) of 21.860 min and other minor peaks.

From the mass spectrometry analysis of rhNGF-118, all the peaks are confirmed to have the same sequence of 118 aminoacids wherein the main peak corresponds to rhNGF 118 without any post-translational modification and the minor peaks correspond to rhNGF-118 having post translational modifications. The molecular weight (MW) of the main peak is of 13252.5 Da (Table 1).

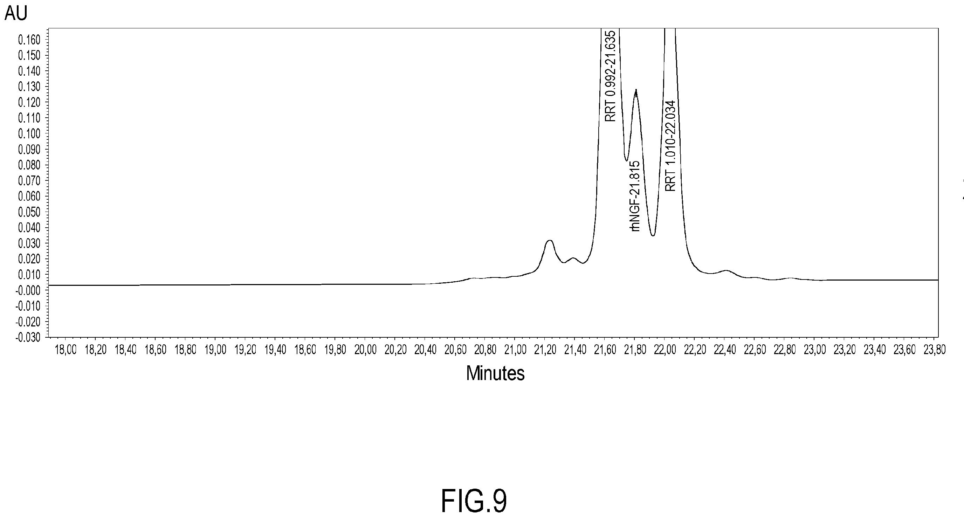

In the Figure 9 the chromatogram of rhNGF-1 is reported. Three main peaks were detected having the RT at 21.635, 21.815 and 22.034 min (see Table 3). From the mass spectrometry analysis, the three main peaks of rhNGF-1 showed sequences of different length, composed of 117, 118, 119 and 120 aminoacids (see Table 1 and Table 3 for the details).

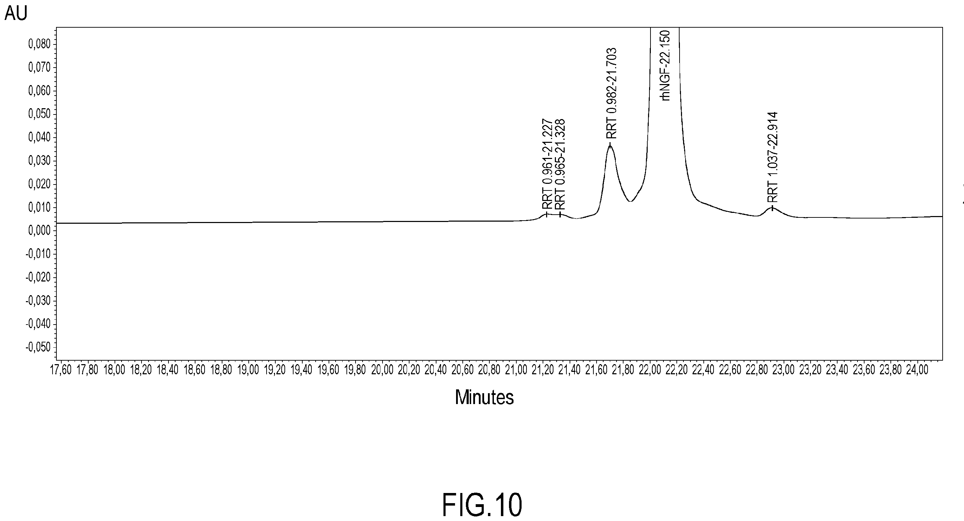

Regarding the rhNGF-2 and rhNGF-3 (Figures 10-11, Tables 4 and 5) they are characterised by a main peak, at the RTs of 22.091 and 22.050 min respectively, both corresponding to a sequence of 117 aminoacids (confirmed by mass spectrometry). The retention time and the composition percentage corresponding to each isoform comprising their related post-translational modified forms, are summarized in Table 1 below (second and third column).

Taken together these results demonstrate that, contrary to rh-NGF-118, the commercial products rhNGF-1, rhNGF-2 and rhNGF-3 do not have a homogeneous composition but contain different forms of rhNGF.

**Table 1**

| Product | RT (min.) | Aminoacid sequence | % Area | MW (Da) |
|---|---|---|---|---|
| rhNGF-118 | 21.860 | NGF118 (SEQ.ID.NO:1) | 91.75 | 13252.5 |
| | See Table 2 | NGF118 (SEQ.ID.NO:1) with post-translational modifications | 8.25 | n.a. |
| rhNGF-1 | 21.635 | NGF120 (SEQ ID NO.2) | 40.08 | 13479.6 |
| | | NGF119 (SEQ ID NO.4) | | 13408.6 |
| | 21.815 | NGF118 (SEQ.ID.NO:1) | 13.91 | 13252.5 |
| | 22.034 | NGF117 (SEQ ID NO.3) | 26.25 | 13096.4 |
| | See Table 3 | Above NGFs with post-translational modifications | 19.76 | n.a. |
| rhNGF-2 | 22.091 | NGF117 (SEQ ID NO.3) | 94.34 | 13096.4 |
| | See Table 4 | NGF117 with post translational modifications | 5.66 | n.a. |
| rhNGF-3 | 22.150 | NGF117 (SEQ ID NO.3) | 92.23 | 13096.4 |
| | See Table 5 | NGF117 with post translational modifications | 7.77 | n.a. |

in which RT means Retention Time (minutes), MW means molecular weight and n.a. means not assessed.

**Table 2: rhNGF-118 (Figure 8)**

| | RRT | ID | RT(min.) | Area | % Area |
|---|---|---|---|---|---|
| 1 | 0.962 | rhNGF-118 with post-translational modification | 21.035 | 28333 | 0.34 |
| 2 | 0.964 | rhNGF-118 | 21.075 | 38142 | 0.45 |
| 3 | 0.982 | rhNGF-118 with post-translational modification | 21.477 | 448992 | 5.33 |
| 4 | 1.000 | rhNGF-118 with post-translational modification | 21.860 | 7310567 | 91.75 |
| 5 | 1.015 | rhNGF-118 with post-translational modification | 22.190 | 75178 | 0.89 |
| 6 | 1.021 | rhNGF-118 with post-translational modification | 22.320 | 82323 | 0.98 |
| 7 | 1.036 | rhNGF-118 with post-translational modification | 22.647 | 4202 | 0.05 |
| 8 | 1.041 | rhNGF-118 with - post-translational modification | 22.757 | 4807 | 0.06 |
| 9 | 1.049 | rhNGF-118 with post-translational modification | 22.925 | 5734 | 0.07 |
| 10 | 1.055 | rhNGF-118 with post-translational modification | 23.065 | 2631 | 0.03 |
| 11 | 1.062 | rhNGF-118 with post-translational modification | 23.222 | 4411 | 0.05 |

in which RT means Retention Time and RRT means Relative Retention Time, relative to the retention time of rhNGF-118.

**Table 3: rhNGF-1 (Figure 9)**

| | RRT | ID | RT (min.) | Area | % Area |
|---|---|---|---|---|---|
| 1 | 0.946 | Related substance | 20.642 | 11024 | 0.18 |
| 2 | 0.951 | Related substance | 20.735 | 24336 | 0.39 |
| 3 | 0.957 | Related substance | 20.868 | 30468 | 0.49 |
| 4 | 0.973 | Related substance | 21.232 | 271995 | 4.41 |
| 5 | 0.981 | Related substance | 21.388 | 109015 | 1.77 |
| 6 | 0.992 | rhNGF-119 and rhNGF-120 | 21.635 | 2472926 | 45.40 |
| 7 | 1,000 | rhNGF-118 | 21.815 | 858522 | 15.76 |
| 8 | 1.010 | rhNGF-117 | 22.034 | 1619783 | 29.74 |
| 9 | 1.027 | Related substance | 22.410 | 69958 | 1.13 |
| 10 | 1.033 | Related substance | 22.533 | 23161 | 0.38 |
| 11 | 1.047 | Related substance | 22.841 | 21619 | 0.35 |

in which RT means Retention Time and RRT means Relative Retention Time relative to the retention time of rhNGF-118. By related substance a post-translational modification of a NGF isoform among rhNGF-117, rhNGF-118, rhNGF-119, rhNGF-120 or mixture thereof, is meant.

**Table 4: rhNGF-2 (Figure 10)**

| | RRT | ID | R T (min.) | Area | % Area |
|---|---|---|---|---|---|
| 1 | 0.961 | rhNGF-117 with post-translational modification | 21.227 | 19451 | 0.33 |
| 2 | 0.965 | rhNGF-117 with post-translational modification | 21.328 | 16818 | 0.28 |
| 3 | 0.982 | rhNGF-117 with post-translational modification | 21.703 | 273260 | 4.58 |
| 4 | 1.000 | rhNGF-117 | 22.091 | 5281364 | 94.34 |
| 5 | 1.037 | rhNGF-117 with post-translational modification | 22.914 | 27691 | 0.46 |

in which RT means Retention Time and RRT means Relative Retention Time relative to the retention time of rhNGF-117.

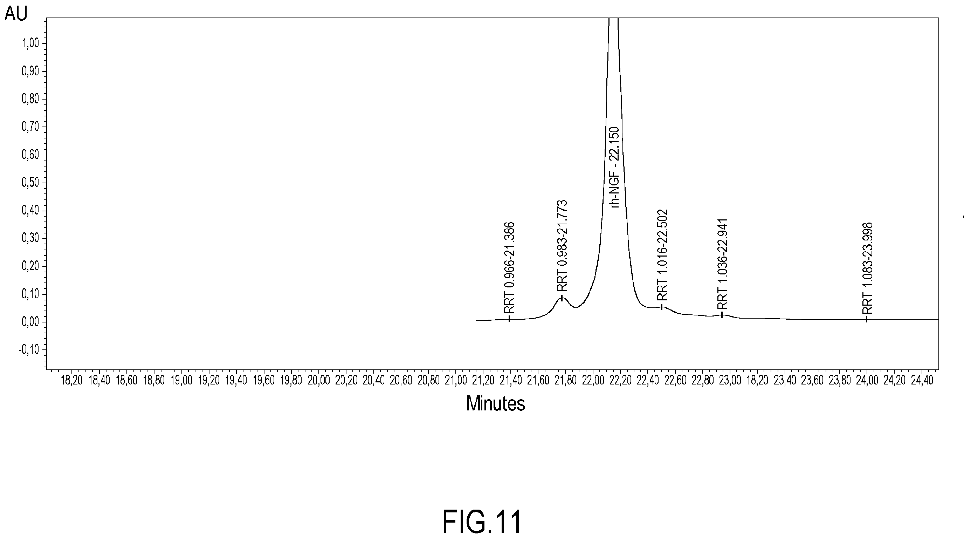

**Table 5: rhNGF-3 (Figure 11)**

| | RRT | ID | RT (min.) | Area | % Area |
|---|---|---|---|---|---|
| 1 | 0.265 | rhNGF-117 with post-translational modification | 5.864 | 31704 | 0.22 |
| 2 | 0.966 | rhNGF-117 with post-translational modification | 21.386 | 109233 | 0.74 |
| 3 | 0.983 | rhNGF-117 with post-translational modification | 21.773 | 869744 | 5.91 |
| 4 | 1.000 | rhNGF-117 | 22.150 | 12947657 | 92.23 |
| 5 | 1.016 | rhNGF-117 with post-translational modification | 22.502 | 81873 | 0.56 |
| 6 | 1.036 | rhNGF-117 with post-translational modification | 22.941 | 44131 | 0.30 |
| 7 | 1.083 | rhNGF-117 with post-translational modification | 23,998 | 7428 | 0,05 |

in which RT means Retention Time and RRT means Relative Retention Time relative to the retention time of rhNGF-117.

As can be seen in Table 1 and in Tables 2 to 5, while rhNGF-118 has a molecular weight that corresponds to that of NGF of SEQ.ID NO.1, the analysed commercial products comprise isoforms of different molecular weight or their admixtures.

While rhNGF-118 consists of 100% of the NGF isoform having the 118 aminoacids sequence of SEQ ID NO.1, possibly with post-translational modifications in a small percentage, the commercial products are characterised by the presence of NGF isoforms having an aminoacid length different from that of 118 aminoacids of SEQ ID NO 1.

In particular, rhNGF-1 comprises a mixture of different isoforms of NGF, having 117 (SEQ ID NO 3), 118 (SEQ ID NO 1), 119 (SEQ ID NO 4) and 120 (SEQ ID NO 2) aminoacids, with a major percentage of the 119 and 120 aminoacid isoforms.

rNGF-2 and rhNGF-3 consist of an isoform of NGF having a sequence of 117 aminoacids (SEQ ID NO.3).

Also the above isoforms having 117 (SEQ ID NO 3), 118 (SEQ ID NO 1), 119 (SEQ ID NO 4) and 120 (SEQ ID NO. 2) aminoacids include a small percentage of post-translational modified protein, for example oxidated or glycated protein.

### Example 2- Proteomic analysis

A proteomic analysis was performed on rhNGF-118 and rhNGF-1 in order to investigate their ability to regulate NGF-responsive intracellular signaling pathways.

To this aim, two human cell lines derived from different regions of the eye, Human Retinal Pigment Epithelium Cells (RPE, hTERT-RPE-1, ATCC CLR-400), Human Corneal Epithelial Cells (HCEC, P10871-IM InnoProt), and one cell line derived from pheochromocytoma of the rat adrenal medulla (PC-12, Ceinge, cod. Art. CF017.04) were used.

NGF signaling was mediated by two main receptors, TrKA and p75, and therefore, before starting the analysis, the expression of these receptors in all cell lines used was assessed by FACS (Fluorescence-activated Cell Sorting).

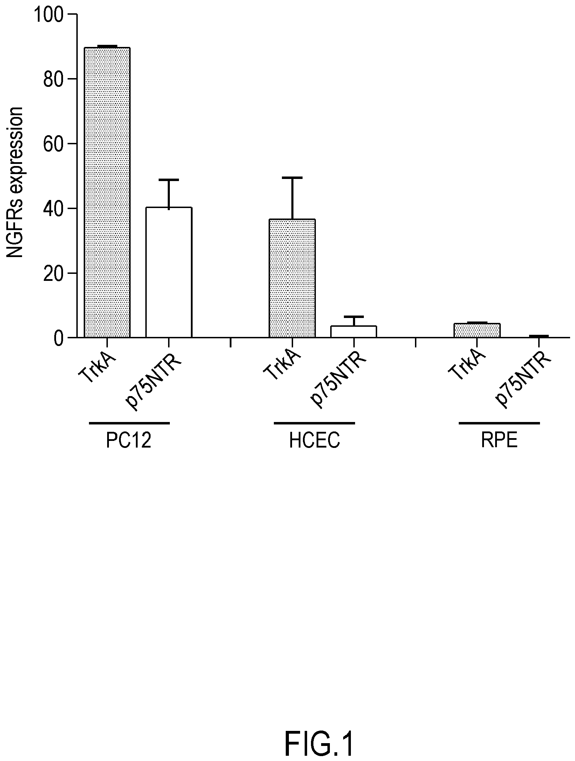

As can be seen in Figure 1, both receptors are expressed in all the cell lines, even though at different amounts.

The cells were treated for 5, 10 or 20 minutes with 50 ng/ml of rhNGF-118 or rhNGF-1 (mainly constituted by rhNGF-119 and rhNGF-120), then lysed and pooled together. 25 to 50 micrograms of pooled lysate proteins from each sample were covalently labelled with biotin. Free biotin molecules were then removed at the completion of the labeling by gel filtration. After blocking non-specific binding sites on the array, an incubation chamber was mounted on to the microarray to allow the loading of 2 samples (normally, one control and one matching treated sample) side by side on the same chip and to prevent mixing of the samples. Following sample incubation, unbound proteins were washed away and the array was probed with anti-biotin antibody labelled with a proprietary fluorescent dye combination captured with a Perkin-Elmer ScanArray Reader laser array scanner. Signal quantification was performed with ImaGene9.0 (BioDiscovery) with predetermined settings for spot segmentation and background correction. The strength of the signal was an indication of the expression level or phosphorylation state of the target protein found in the cells, taken with duplicate measurements.

Protein changes among the three cell lines after treatment with each rhNGF and then, protein changes among the different rhNGF treatments in each individual cell line were analyzed.

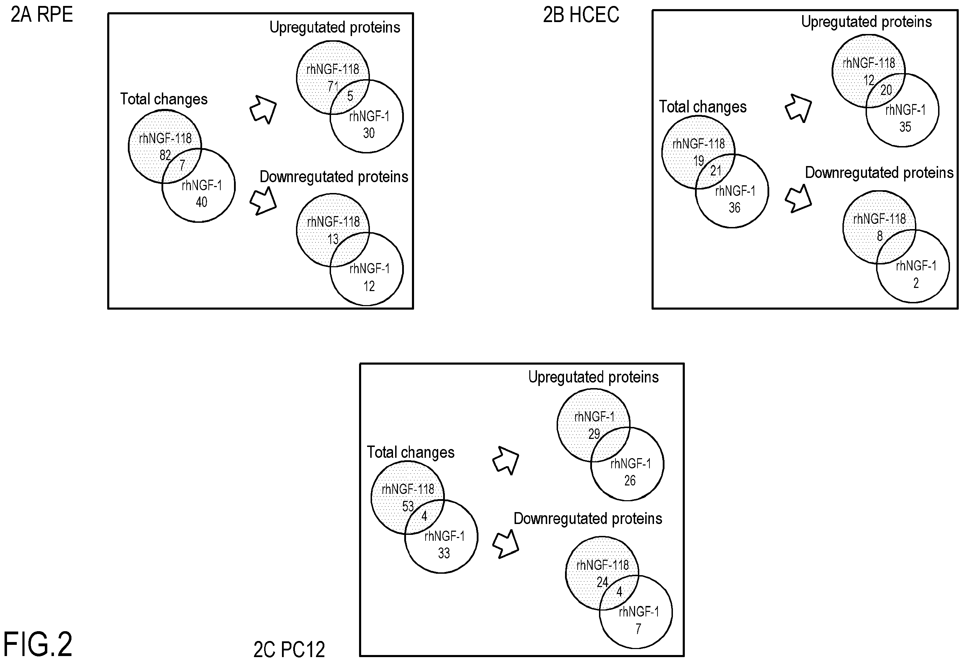

The data so obtained show that, in accordance with the different levels of expression identified for the two main NGF receptors (TrKA and p75) in the three cell lines, both the tested NGFs regulate proteins in a cell-dependent manner (Figure 2). Furthermore, although some proteins are identically modulated by the different tested rhNGFs, other proteins show a pattern of modulation that was specific for each rhNGF (Figure 2).

On the basis of the regulated proteins, we could identify three main intracellular pathways that are modulated by NGF: apoptosis, survival/proliferation and differentiation.

The results of the analysis show the ability of rhNGF-118 but not of rhNGF-1 to promote pathways associated to cell proliferation and survival and to inhibit the activation of pathways associated with apoptosis, in line with the findings shown in Table 6 below:

**Table 6**

| | RPE | | HCEC | | PC12 | |
|---|---|---|---|---|---|---|
| | rhNGF-118 | rhNGF-1 | rhNGF-118 | rhNGF-1 | rhNGF-118 | rhNGF-1 |
| p53 | - | | | + | | + |
| MDM2 | | + | + | | + | |
| Jun | | + | | + | | |
| Pim2/3 | + | | | | + | |

| | | | | | | |
|---|---|---|---|---|---|---|
| wherein (+) indicates upregulation and (-) downregulation. | | | | | | |

From Table 6, it clearly appears that rhNGF-118 unlike rhNGF-1 downregulates p53, both reducing p53 expression (see RPE) and inducing MDM2 expression, an important negative regulator of p53 (see HCEC and PC12). In addition, rhNGF-118 was able to upregulate Pim2 and Pim3 proteins that overall show anti-apoptotic effect inactivating BAD. On the other hand, rhNGF-1 treatments resulted in directly upregulation of p53 (see HCEC and PC12) and upregulation of Jun, that can contribute to the apoptotic effect. The results obtained suggest that the two tested NGFs activate different downstream pathways in the same cell. Overall, treatment of cells with rhNGF-118 results in a modulation of proteins that points toward a reduction of apoptosis and a promotion of cell proliferation. On the contrary, treatment of cells with rhNGF-1 results in changes of proteins in line with a potentially increased apoptosis. These data suggest that rhNGF-118 exerts a stronger activity over the TrKA-mediated pathways, while rhNGF-1 activates pathways mediated by p75^{NTR}, as shown in the summary Table 7 below:

**Table 7**

| Cell line | Process | Receptor involved | rhNGF-118 | rhNGF-1 |
|---|---|---|---|---|
| RPE | Apoptosis | P75 ^{NTR} | Inhibited | Activated |
| | Proliferation | TrKA | Activated | Unchanged |
| HCEC | Apoptosis | P75 ^{NTR} | Inhibited | Activated |
| | Proliferation | TrKA | Activated | Activated |
| PC12 | Apoptosis | P75^{NTR} | Inhibited | Activated |
| | Proliferation | TrKA | Activated | Unchanged |
| | Differentiation | P75^{NTR} | Unchanged | Inhibited |
| | | TrKA | Activated | Unchanged |

As can be seen from Table 7, in RPE, HCEC and PC12 cells, treatment with rhNGF-118 inhibits apoptosis and promotes proliferation, while treatment with rhNGF-1 activates apoptosis and does not change or promotes proliferation. The two NGFs also have opposite effects on PC12 cell differentiation.

rhNGF-118 trend to inhibit p53-dependent apoptosis supports a pivotal protective role of this molecule against eye pathological conditions of the retina and cornea.

In retina, corneal and conjunctival cells, p53 promotes apoptosis and is associated with several eye pathological conditions, while reduction of its expression and activation protects retina and corneal cells from death. In a previous study, upregulation of p53 was found in the human RPE cell line ARPE-19 after incubation with A2E (the major RPE lipofuscin fluorophore and mediator of light damage to RPE cells) and exposure to high-energy visible (HEV) light. Instead, transfection with siRNA against TP53 before exposure to HEV light protected the cells and reduced apoptosis, demonstrating the crucial role of p53 in bright light-mediated damage. Furthermore, age-related macular degeneration (AMD), a leading cause of blindness in the elderly, is often associated with lipofuscin accumulation in the RPE cells and RPE cell death. Thus, given the role of p53 in lipofuscin-associated cell death *in vitro* and the fact that the inhibition of the p53 inhibitor Mdm2 has been shown to sensitize human RPE cells to apoptosis, it is reasonable to assume that RPE cell death in AMD involves the p53 pathway (Vuon et al., Invest Ophthalmol Vis Sci 2012, .53, 1362-1371).

### Example 3-Analysis of effect on apoptosis

### a) Effect on apoptosis in normal conditions

To confirm the different ability of the rhNGF isoforms to inhibit apoptosis, as suggested by the proteomic data, the ability of NGFs to induce Caspase 3/7, a commonly used marker of apoptosis, was analysed in HCEC and RPE cells by using an Incucyte^{®} Caspase-3/7 Activation assay.

The Incucyte^{®} Caspase-3/7 Reagents freely cross the cell membrane of cells. In apoptotic cells, they are cleaved by activated caspase-3/7 to release DNA-binding fluorescent label, thus allowing visualization of these cells as fluorescent nuclei.

HCEC and RPE cells, described above, were not treated (NT) or treated with PBS (PBS), formulation buffer (FB), rhNGF-118 (50 ng/ml) or rhNGF-1 (50 ng/ml) and induction of Caspase 3/7 was assessed in each sample after 30 minutes and 24 hours.

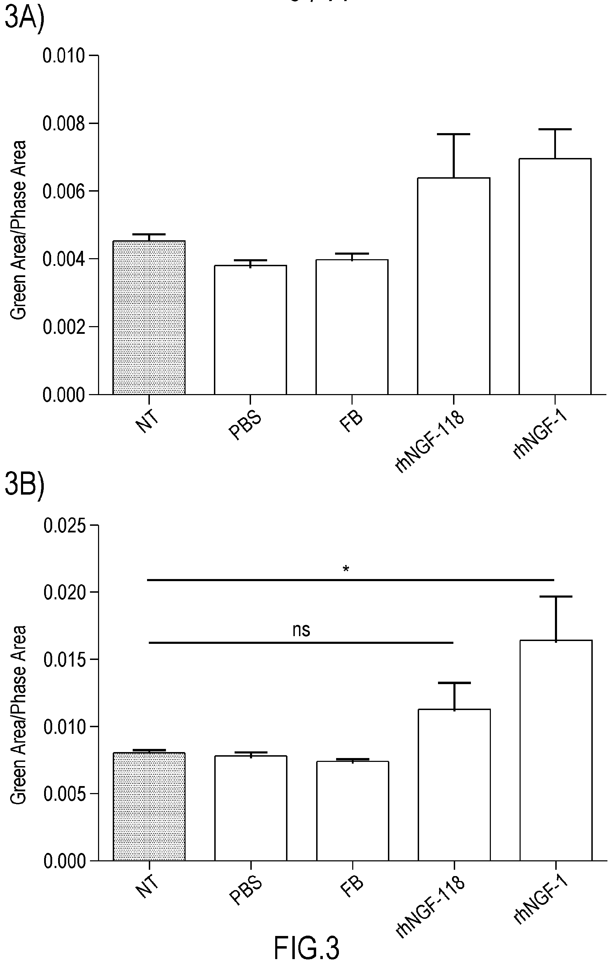

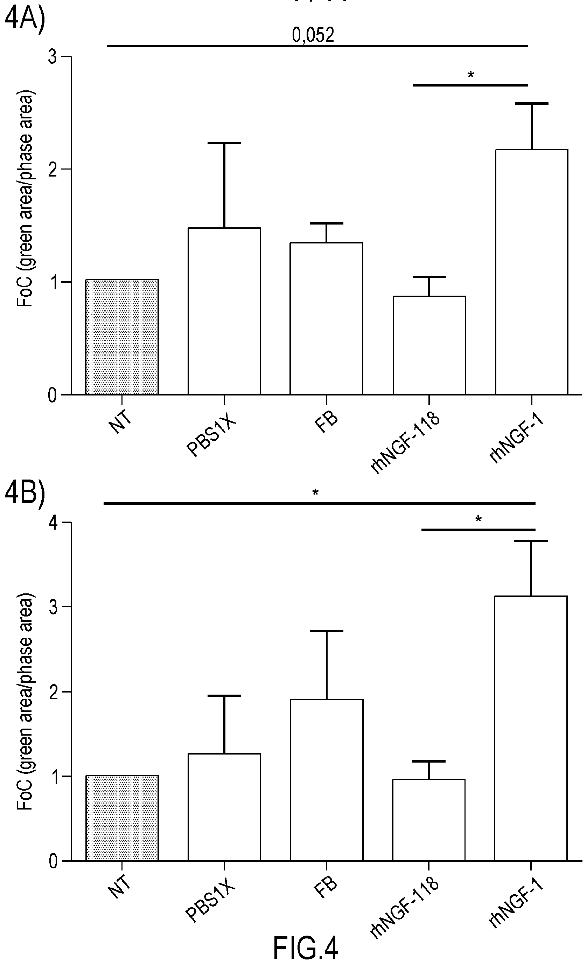

As can be seen in Figures 3 and 4, while treatment of the cells with rhNGF-118 did not result in any significant induction of Caspase 3/7, rhNGF-1 caused an upregulation of this apoptotic marker, particularly evident at 24 hours after treatment, compared to the untreated control.

### b) Effect on apoptosis under oxidative stress conditions

### i) Apoptosis induced by tert-butvlhvdroperoxide (tBHP)

RPE cells were incubated for 24 hours with tert-butylhydroperoxide (tBHP) 10µM, a stress commonly used to induce apoptosis in cells, in the presence of Formulation Buffer (FB), rhNGF-118 (50 ng/ml), rhNGF-2 (50 ng/ml) or rhNGF-3 (50 ng/ml) and the activation of Caspase-3/7 was evaluated.

After 24 hours of tBHP exposure, cells treated with rhNGF-118 showed a significant reduction of apoptosis compared to the control treated with FB. On the contrary, treatment with both rhNGF-2 and rhNGF-3 did not reduce the tBHP-induced apoptosis that resulted instead significantly increased compared to rhNGF-118 (Figure 5).

### ii) Apoptosis induced by H₂O₂

To further assess the antiapoptotic activity of rhNGF-118, 100 µM H₂O₂, a stress-inducing agent commonly used to induce apoptosis in cells, was added to the culture medium of RPE cells, in the absence or in the presence of 50 ng/ml of rhNGF-118 or rhNGF-1. When applicable, the tested NGF was added simultaneously to the H₂O₂. As controls, cells untreated or treated only with PBS, H₂O₂, rhNGF-118 or rhNGF-1 were used. The apoptotic signal in each sample was measured after 30 minutes (Figure 6A) and 24 hours (Figure 6B) of incubation and the fold increase in apoptosis relative to the untreated sample was calculated.

As expected, H₂O₂ treatment resulted in increased apoptotic levels. Surprisingly, this increase was prevented when the cells were incubated with rhNGF-118, but not with rhNGF-1 (Figure 6A and 6B). On the contrary, when rhNGF-1 was present in the cells, a greater level of apoptosis was observed compared to the control cells treated only with H₂O₂.

Together these data confirm the proteomic analysis results and show that rhNGF-118 prevents apoptosis in corneal and retinal epithelial cells. More importantly, these results also point toward a protective role of rhNGF-118 against stress, such as the one induced by H₂O₂.

### Example 4

### Effect on neuronal differentiation

Next, we compared rhNGF-118 and rhNGF-1 in an *in vitro* functional assay of neuronal differentiation using PC12 cells. This is a highly validated model to study neuronal differentiation *in vitro,* as PC12 cells respond to nerve growth factor (NGF) and exhibit a typical phenotype of neuronal cells sending out neurites.

PC12 cells express high levels of both TrKA and p75 receptors (Figure 1).

PC12 cells were untreated or treated with each rhNGF for 6 days, at different concentrations (25 and 50 ng/ml). Larger cell bodies and elaboration of an extensive network of neurites was observed in the treated cells compared to the untreated cells, which increased in a dose dependent manner. In the absence of NGF, cells were relatively small rounded and had no visible neurites.

Neurite length was measured by using NeuroTrack software that analyses phase contrast images acquired using the IncuCyte Live-Cell Analysis. Cell bodies were segmented from background based on texture and/or brightness and neurites (linear features) were segmented based on width and brightness. The total neurites length was normalized to image area (mm/mm²).

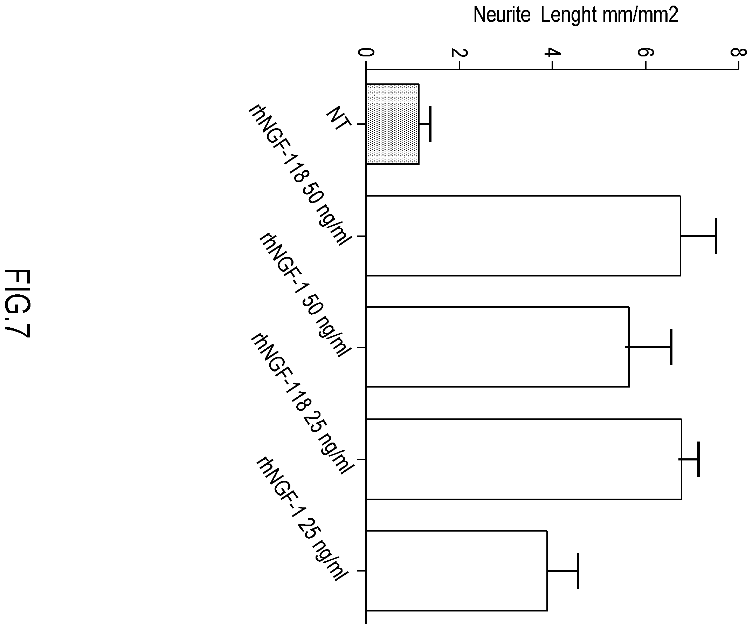

As can be seen from Figure 7, treatment with rhNGF-118 resulted in an increased neuritogenesis compared to rh-NGF-1, especially at 25 ng/ml.

### Example 5

### Binding to TrKA and p75 receptors

One possible reason for the different biological properties of the tested rhNGF isoforms could be a different binding affinity for the receptors TrKA and p75^{NTR}.

We therefore performed a surface plasmon resonance (SPR) analysis by immobilizing TrKA or p75^{NTR} receptors on the sensor surface and then injecting the different rhNGFs isoforms. Affinity values were obtained from measurement of steady-state binding levels. The model of Steady State 1:1 calculates the equilibrium dissociation constant Kd for 1:1 interaction from a plot of steady state binding levels against analyte concentration.

The results so obtained are shown in the Table 8 below:

**Table 8**

| NGF | KDeq | |
|---|---|---|
| | TrKa | P75 |
| rhNGF-118 | 4.332 nM | 7.103 nM |
| rhNGF-1 | 3.647 nM | 5.0565 nM |
| rhNGF-2 | 3.852 nM | 15.70 nM |
| rhNGF-3 | 5.999 nM | 39.15 nM |

As can be seen from the data, rhNGF-2 and rhNGF-3 show a much higher affinity for p75^{NTR}, while both rhNGF-118 and rhNGF-1 show very similar affinity for TrKA and p75^{NTR} receptors.

Since we could not observe any difference in the binding affinity of rhNGF-118 and rhNGF-1, we focused on the kinetic of the binding of these two NGFs to the receptors. From the analysis of the sensorgrams, by BIA evaluation software, the kinetic parameters (Kon = association rate constant, Koff = dissociation rate constant) and the binding (Kd) value as the ratio of kinetic rate constants (Koff /Kon) were assessed.

The results obtained are shown in the Table 9 below:

**Table 9**

| NGF | kd_{cin} | |
|---|---|---|
| | TrKa | P75 |
| rhNGF-118 | Kon=1.666x10⁶(1/Ms) | Kon=6.5x10¹⁰ (1/Ms) |
| | Koff=7.5x10⁻⁴ (1/s) | Koff=121.825 (1/s) |
| | kd=0.38 (nM) | kd=2.6 (nM) |
| rhNGF-1 | Kon=8.3x10⁶ (1/Ms) | Kon=8.3x10⁶ (1/Ms) |
| | Koff=6.1x10⁻⁴ (1/s) | Koff=0.01113 (1/s) |
| | kd=0.0792 (nM) | kd=1.3255 (nM) |

As can be seen from the data above, the two NGFs show different kinetics of binding to the two receptors.

In particular, rhNGF-118 binds to and dissociates from p75^{NTR} very rapidly, while rhNGF-1 dissociates from p75^{NTR} much slower.

Based on these data, we conclude that rhNGF-118 dissociates quickly from p75^{NTR} and it is therefore more available for binding TrKA and activating its downstream signalling. On the other hand, the slow dissociation of rhNGF-1 from p75^{NTR} could explain the prolonged downstream p75^{NTR}-mediated effects.

### SEQUENCE LISTING

SEQ ID No. 1
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVR

SEQ ID No. 2
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRRA

SEQ ID No. 3
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AV

SEQ ID No. 4
SSSHPIFHRGEFSVCDSVSVWVGDKTTATDIKGKEVMVLGEVNINNSVFKQYFFETKC
RDPNPVDSGCRGIDSKHWNSYCTTTHTFVKALTMDGKQAAWRFIRIDTACVCVLSRK
AVRR

## Claims

1. A NGF for use in the prevention and/or treatment of an ocular pathology selected from retinopathies, corneal pathologies, optic neuropathies, conjunctival pathologies, limbal stem cell deficiency and in the prevention of allograft rejection in corneal transplantation, in which said NGF comprises more than 50% by weight of the NGF isoform of SEQ ID NO 1 relative to the total weight of all NGF isoforms possibly comprised in said NGF.

2. The NGF for use according to claim 1 in which said NGF comprises at least 60%, more preferably at least 70%, even more preferably at least 80%, more preferably at least 90%, even more preferably at least 95%, even more preferably at least 98%, even more preferably at least 99%, even more preferably 100% by weight of the NGF isoform of SEQ ID NO 1 relative to the total weight of all NGF isoforms possibly comprised in said NGF.

3. The NGF for use according to claims 1 or 2 in which said NGF isoforms possibly comprised in said NGF comprise NGF isoforms of SEQ ID NO 1, 2, 3, 4 or admixtures thereof.

4. The NGF for use according to any one of the previous claims in which said NGF comprises NGF isoforms of SEQ ID NO 2, 3, 4 or their admixtures in total weight lower than 20%, more preferably lower than 10%, even more preferably lower than 5% by weight, relative to the total weight of all NGF isoforms possibly comprised in said NGF.

5. The NGF for use according to any one of the previous claims in which said NGF comprises NGF isoforms of SEQ ID NO 2, 3, 4 or their admixtures in total weight lower than 2%, preferably lower than 1% by weight, relative to the total weight of all NGF isoforms possibly comprised in said NGF.

6. The NGF for use according to any one of the previous claims in which said NGF isoform of SEQ ID NO 1 does not include more than 15%, preferably more than 10% or than 5% by weight of post-translational modifications in total, more preferably it does not include any post-translational modification.

7. The NGF for use according to any one of the previous claims in which said retinopathies are selected from diabetic retinopathy, retinopathy of prematurity, retinal vascular occlusions, phototoxic retinopathy, retinal detachment, age-related macular degeneration, macular degeneration, macular atrophy, macular hole, macular edema and epiretinal membrane.

8. The NGF for use according to any one of claims 1 to 6 in which said corneal pathologies are selected from keratoconus, phototoxic keratopathy, persistent epithelial defects, corneal ulcers, corneal dystrophies and degeneration and keratoconjunctivitis sicca.

9. The NGF for use according to any one of claims 1 to 6 in which said optic neuropathies are selected from glaucoma and ischemic, degenerative, traumatic, inherited and congenital neuropathies.

10. A pharmaceutical composition comprising the NGF for use according to any one of claims 1 to 9 in a therapeutically effective amount and at least one pharmaceutically acceptable excipient.

11. The composition according to claim 10 that is a pharmaceutical composition for ophthalmic use.

12. The composition according to claim 11 that is an ophthalmic liquid composition, preferably an ophthalmic aqueous liquid composition.

13. The composition according to claim 12 that is an aqueous eye drop composition for topical administration to the anterior segment of the eye.

14. The composition according to claim 13, comprising said NGF is in a concentration ranging from about 0.0001% to about 0.5% w/v, more preferably from about 0.001% to about 0.1% w/v, most preferably of about 0.002% w/v of the aqueous composition.

15. The composition according to claim 11 for administration to the posterior segment of the eye.


SEQUENCE LISTING

<110> Dompè Farmaceutici SpA

<120> NGF ISOFORM FOR USE IN THE TREATMENT OF OCULAR PATHOLOGIES

<130> 248EP

<160> 4

<170> BiSSAP 1.3.6

<210> 1
<211> 118
<212> PRT
<213> Escherichia coli

<220>
<223> Recombinant human NGF

<400> 1
Ser Ser Ser His Pro Ile Phe His Arg Gly Glu Phe Ser Val Cys Asp
1               5                   10                  15
Ser Val Ser Val Trp Val Gly Asp Lys Thr Thr Ala Thr Asp Ile Lys
            20                  25                  30
Gly Lys Glu Val Met Val Leu Gly Glu Val Asn Ile Asn Asn Ser Val
        35                  40                  45
Phe Lys Gln Tyr Phe Phe Glu Thr Lys Cys Arg Asp Pro Asn Pro Val
    50                  55                  60
Asp Ser Gly Cys Arg Gly Ile Asp Ser Lys His Trp Asn Ser Tyr Cys
65                  70                  75                  80
Thr Thr Thr His Thr Phe Val Lys Ala Leu Thr Met Asp Gly Lys Gln
                85                  90                  95
Ala Ala Trp Arg Phe Ile Arg Ile Asp Thr Ala Cys Val Cys Val Leu
            100                 105                 110
Ser Arg Lys Ala Val Arg
        115

<210> 2
<211> 120
<212> PRT
<213> Artificial Sequence

<220>
<223> Recombinant human NGF

<400> 2
Ser Ser Ser His Pro Ile Phe His Arg Gly Glu Phe Ser Val Cys Asp
1               5                   10                  15
Ser Val Ser Val Trp Val Gly Asp Lys Thr Thr Ala Thr Asp Ile Lys
            20                  25                  30
Gly Lys Glu Val Met Val Leu Gly Glu Val Asn Ile Asn Asn Ser Val
        35                  40                  45
Phe Lys Gln Tyr Phe Phe Glu Thr Lys Cys Arg Asp Pro Asn Pro Val
    50                  55                  60
Asp Ser Gly Cys Arg Gly Ile Asp Ser Lys His Trp Asn Ser Tyr Cys
65                  70                  75                  80
Thr Thr Thr His Thr Phe Val Lys Ala Leu Thr Met Asp Gly Lys Gln
                85                  90                  95
Ala Ala Trp Arg Phe Ile Arg Ile Asp Thr Ala Cys Val Cys Val Leu
            100                 105                 110

Ser Arg Lys Ala Val Arg Arg Ala
        115                 120

<210> 3
<211> 117
<212> PRT
<213> Artificial Sequence



<220>
<223> Recombinant human NGF

<400> 3
Ser Ser Ser His Pro Ile Phe His Arg Gly Glu Phe Ser Val Cys Asp
1               5                   10                  15
Ser Val Ser Val Trp Val Gly Asp Lys Thr Thr Ala Thr Asp Ile Lys
            20                  25                  30
Gly Lys Glu Val Met Val Leu Gly Glu Val Asn Ile Asn Asn Ser Val
        35                  40                  45
Phe Lys Gln Tyr Phe Phe Glu Thr Lys Cys Arg Asp Pro Asn Pro Val
    50                  55                  60
Asp Ser Gly Cys Arg Gly Ile Asp Ser Lys His Trp Asn Ser Tyr Cys
65                  70                  75                  80
Thr Thr Thr His Thr Phe Val Lys Ala Leu Thr Met Asp Gly Lys Gln
                85                  90                  95
Ala Ala Trp Arg Phe Ile Arg Ile Asp Thr Ala Cys Val Cys Val Leu
            100                 105                 110
Ser Arg Lys Ala Val
        115

<210> 4
<211> 119
<212> PRT
<213> Artificial Sequence



<220>
<223> Recombinant human NGF

<400> 4
Ser Ser Ser His Pro Ile Phe His Arg Gly Glu Phe Ser Val Cys Asp
1               5                   10                  15
Ser Val Ser Val Trp Val Gly Asp Lys Thr Thr Ala Thr Asp Ile Lys
            20                  25                  30
Gly Lys Glu Val Met Val Leu Gly Glu Val Asn Ile Asn Asn Ser Val
        35                  40                  45
Phe Lys Gln Tyr Phe Phe Glu Thr Lys Cys Arg Asp Pro Asn Pro Val
    50                  55                  60
Asp Ser Gly Cys Arg Gly Ile Asp Ser Lys His Trp Asn Ser Tyr Cys
65                  70                  75                  80
Thr Thr Thr His Thr Phe Val Lys Ala Leu Thr Met Asp Gly Lys Gln
                85                  90                  95
Ala Ala Trp Arg Phe Ile Arg Ile Asp Thr Ala Cys Val Cys Val Leu
            100                 105                 110
Ser Arg Lys Ala Val Arg Arg
        115